(19) Europäisches Patentamt
European Patent Office
Office européen des brevets

(11) Publication number: **0 160 618 B1**

(12) # EUROPEAN PATENT SPECIFICATION

(45) Date of publication of patent specification: **14.08.91**

(51) Int. Cl.[5]: **C07D 233/66**, C07D 233/74, A61K 31/415

(21) Application number: **85810100.9**

(22) Date of filing: **08.03.85**

(54) **Hydantoin derivatives and pharmaceutical compositions containing them.**

(30) Priority: **08.03.84 JP 45278/84**
**15.11.84 JP 241439/84**

(43) Date of publication of application:
**06.11.85 Bulletin 85/45**

(45) Publication of the grant of the patent:
**14.08.91 Bulletin 91/33**

(84) Designated Contracting States:
**AT BE CH DE FR GB IT LI LU NL SE**

(56) References cited:
**DE-A- 2 346 535**
**US-A- 3 696 198**
**US-A- 4 227 005**
**US-A- 4 230 709**
**US-A- 4 230 869**

**J. Het. Chem. (1970) 7, 1289-1293**

**Chem. Ber. (1921) 54, 1829-1833**

**J. Org. Chem. (1979) 44, 3769-3778**

**Tetrahedron (1977) 33, 1191-1196**

(73) Proprietor: **NIPPON ZOKI PHARMACEUTICAL CO. LTD.**
**10, 2-chome Hirano-machi Higashi-ku**
**Osaka-shi Osaka-fu(JP)**

(72) Inventor: **Ienaga, Kazuharu, c/o Nippon Zoki Pharm. Co.Ltd.**
**Inst.of Bio-Active Science, 422-1 Aza Kawakitayama**
**Kinashi, Yashiro-cho, Katoh-gun, Hyogo(JP)**
Inventor: **Nakamura, Ko, c/o Nippon Zoki Pharm. Co.Ltd.**
**Inst.of Bio-Active Science, 422-1 Aza Kawakitayama**
**Kinashi, Yashiro-cho, Katoh-gun, Hyogo(JP)**

(74) Representative: **Kerr, Andrew**
**Postfach 122 Finkelerweg 44**
**CH-4144 Arlesheim BL(CH)**

**EP 0 160 618 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Synthesis (1984), 270-271

Chem. Abstr. (1983) 98, 53896b

Chem. Abstr. (1984) 101, 181063v

J. Het. Chem. (1981) 18, 1629-1633

## Description

The present invention relates to hydantoin derivatives, pharmaceutically acceptable salts thereof and pharmaceutical compositions containing them as an active ingredient.

Although the oral sulfonylureas and biguanides having hypoglycemic activity have previously been used for the treatment of diabetes, they have often given rise to side effects such as excessive hypoglycemia and lactic acidosis. On the other hand, insulin, a well-known antidiabetic, can only be administered intravenously.

It has now been found that certain hydantoin derivatives and pharmaceutically acceptable salts thereof possess excellent hypoglycemic, hypolipidemic and diuretic effects as well as low toxicity and increased safety.

An object of the present invention is to provide orally administrable hydantoin derivatives and pharmaceutically acceptable salts thereof having hypoglycemic, hypolipidemic and diuretic activity as well as low toxicity, diminished side effects and good safety properties. These derivatives and salts are useful for preparing pharmaceutical compositions.

US-A-4 230 709 discloses the use of compounds of formula 11 where X is H and $R_1$, $R_2$, and $R_3$ are $C_{1-6}$ alkyl for treating hypersensitivity and asthma, and US-A-3 696 198 the use of compounds of formula 11 where X is H, $R_1$ is H and $R_2$ and $R_3$ are $C_{1-4}$ alkyl for enhancing oxygen utilisation.

The following documents:

J. Het. Chem. (1970) 7, 1289-1293
Chem. Ber. (1921) 54, 1829-1833
J. Org. Chem. (1979) 44, 3769-3778
Tetrahedron (1977) 33, 1191-1196
DE-A-2 346 535
Synthesis (1984) 270-271
Chem. Abstr. (1983) 98, 53896b
Chem. Abstr. (1984) 101, 181063v
J. Het. Chem. (1981) 18, 1629-1633
disclose non-medical uses of a variety of compounds of formula 1 where $R_1$ is hydrogen or methyl.

Accordingly the present invention is confined as regards pharmaceutical compositions to the compounds of formula 1, and as regards novel compounds per se to compounds of formula 1 where $R_1$ has at least 2 carbon atoms.

Thus the invention provides according to one aspect a hydantoin derivative of the general formula (I)

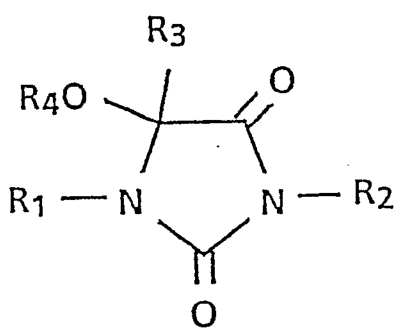

(I)

wherein $R_1$ represents a straight or branched chain alkyl group having from 2 to 20 carbon atoms or a cycloalkyl group having from 3 to 8 carbon atoms, and each of $R_2$, $R_3$ and $R_4$, which may be the same or different, represents hydrogen or a straight or branched chain alkyl group having from 1 to 20 carbon atoms or a cycloalkyl group having from 3 to 8 carbon atoms,
and pharmaceutically acceptable salts thereof.

The invention provides according to another aspect a pharmaceutical composition containing a compound of the general formula (I) (above) wherein each of $R_1$, $R_2$, $R_3$ and $R_4$, which may be the same or different, represents hydrogen, an alkyl group having from 1 to 20 carbon atoms or a cycloalkyl group having from 3 to 8 carbon atoms,
and pharmaceutically acceptable salts thereof, together with a pharmaceutically acceptable carrier or diluent.

The invention provides according to a third aspect the use of hydantoin derivatives of the general

formula (II)

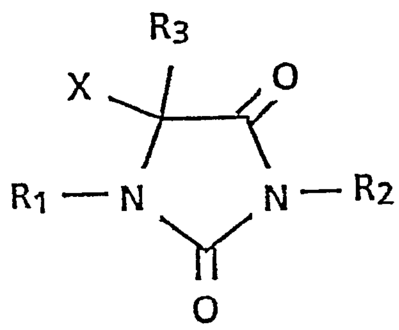

(II)

wherein X represents a hydrogen atom or a group of the formula $-OR_4$, and each of $R_1$, $R_2$, $R_3$ and $R_4$, which may be the same or different, represents hydrogen, an alkyl group having from 1 to 20 carbon atoms or a cycloalkyl group having from 3 to 8 carbon atoms,
and pharmaceutically acceptable salts thereof for the manufacture of hypoglycemic agents, hypolipidemic agents or diuretics.

In the second and third aspects of the invention, $R_4$ represents a hydrogen atom, an alkyl group, preferably a straight or branched alkyl group having 1 to 20 carbon atoms, such as methyl, ethyl, propyl, iso-propyl, butyl, iso-butyl, sec-butyl, tert-butyl, pentyl, iso-pentyl, neo-pentyl, tert-pentyl, hexyl, iso-hexyl, dimethylbutyl, heptyl, octyl, nonyl, decyl or stearyl, or a cycloalkyl group, preferably having 3 to 8 carbon atoms such as cyclopropyl, cyclobutyl, cyclopentyl, cyclohexyl, cycloheptyl or cyclooctyl. Each of $R_1$, $R_2$ and $R_3$ which may be the same or different represents a hydrogen atom, an alkyl group, preferably a straight or branched alkyl group having 1 to 20 carbon atoms, such as methyl, ethyl, propyl, iso-propyl, butyl, iso-butyl, sec-butyl, tert-butyl, pentyl, iso-pentyl, neo-pentyl, tert-pentyl, hexyl, iso-hexyl, dimethylbutyl, heptyl, octyl, nonyl, decyl or stearyl, or a cycloalkyl group, preferably having 3 to 8 carbon atoms such as cyclopropyl, cyclobutyl, cyclopentyl, cyclohexyl, cycloheptyl or cyclooctyl.

Preferred compounds of the present invention are set out below:
5-hydroxy-1-methylhydantoin, 5-hydroxy-3-methylhydantoin, 5-hydroxy-1-ethylhydantoin, 5-hydroxy-1-butylhydantoin, 5-hydroxy-1-tert.-butylhydantoin, 5-hydroxy-1-hexylhydantoin, 5-hydroxy-1-decylhydantoin, 5-hydroxy-1-stearylhydantoin, 5-hydroxy-1-cyclopentylhydantoin, 5-hydroxy-1-cyclohexylhydantoin, 5-hydroxy-1,3-dimethylhydantoin, 5-hydroxy-1,5-dimethylhydantoin, 5-hydroxy-3,5-dimethylhydantoin, 5-hydroxy-1-(1,3-dimethylbutyl)hydantoin, 5-hydroxy-1-cyclohexyl-3-methylhydantoin, 5-hydroxy-1,3-dicyclohexylhydantoin, 5-methoxy-1-methylhydantoin, 5-methoxy-3-methylhydantoin, 5-ethoxy-1-methylhydantoin, 5-butoxy-3-methylhydantoin, 5-methoxy-1-cyclohexylhydantoin, 5-methoxy-3-cyclohexylhydantoin or 1-methylhydantoin.

The pharmaceutically acceptable salts of the compounds of the above-mentioned general formula (I) may be salts with alkali metals such as lithium, sodium or potassium, with alkaline earth metals such as calcium or magnesium, with other metals such as aluminium or silver, or with organic bases such as ammonia or organic amines.

These salts may be produced from the free hydantoin derivatives or converted with each other in a conventional manner.

When optical isomers exist in any of the compound of the present invention, the present invention includes the dl-, l- and d-isomers.

The hydantoin derivatives of the present invention may be prepared by any convenient method, e.g. by one of the conventional methods set out below:-

(1) In the first place, a glyoxylic acid is conventionally esterified. That is, a glyoxylic acid is reacted with an alcohol or 2-methoxyethanol, with the produced water being removed, in the presence of an organic acid catalyst such as p-toluenesulfonic acid or camphorsulfonic acid in an aprotic solvent such as benzene, toluene, xylene or carbon tetrachloride at room temperature or on heating at an appropriate temperature or under reflux, for several hours to one day. Produced glyoxylic acid ester or o-alkylglyoxylic acid ester (glyoxylic acid ester alcoholate) is, without being isolated or after purified, reacted at room temperature or heated under reflux for 1 hour to several days with N-alkylurea, N-cycloalkylurea, N,N'-dialkylurea or N,N'-dicycloalkylurea in an appropriate solvent such as water, acetic acid or alcohol such as butanol or mixtures thereof to give compounds of the present invention represented by the general formula (I).

It is also possible to carry out the above-mentioned reaction in the same way using as materials each of glyoxylic acid, o-alkylglyoxylic acid ester (glyoxylic acid ester alcoholate) or α-ketocarbonic acid such as pyruvic acid and each of N-alkylurea, N-cycloalkylurea, N,N'-dialkylurea or N,N'-dicycloalkylurea.

(2) The compounds of the present invention wherein $R_4$ is an alkyl or cycloalkyl group may be produced by a conventional O-alkylation process as follows:

The hydantoin derivative is reacted with p-toluenesulfonyl chloride or mesyl chloride to introduce a removable residue into hydroxy group at the 5-position in the presence of an organic base such as lower alkylamines or alkali metal alkoxides in an appropriate solvent not inhibiting the reaction. During or after the reaction, the resultant product is reacted with the alcohol corresponding to the $R_4$ of the intended hydantoin derivative to give the compound of the present invention. This O-alkylation may be carried out at room temperature or on heating at an appropriate temperature or under reflux, for several hours to several days.

(3) The compounds of the present invention may also be produced by a conventional N-alkylation, e.g. as follows:-

The hydantoin derivative is reacted with a halogenated alkyl, halogenated cycloalkyl or dialkylsulfuric acid such as dimethylsulfuric acid, p-toluenesulfonic acid alkyl ester or p-toluenesulfonic acid cycloalkyl ester in the presence of a base such as lower alkyl amines, alkali metal alkoxides or hydroxyalkyl metals in an appropriate solvent which does not inhibit the reaction such as absolute alcohol or dimethyl sulfoxide. This N-alkylation may be carried out at room temperature or on heating at an appropriate temperature for several hours to several days.

The resultant compounds of the present invention are purified by usual methods such as distillation, chromatography and recrystallization. They are identified by elemental analysis, melting point, IR, NMR, UV and mass spectrum.

The following examples, which are illustrative, describe the preparation of the compounds of the present invention.

Example 1.

15.0 g of glyoxylic acid n-butyl ester monohydrate and 7.4 g of N-methylurea were refluxed in 80% aqueous solution of acetic acid for 1 hour. After the solvent was removed by distillation, a small amount of methanol was added to the residue and the insoluble matter was filtered off. The filtrate was then evaporated to dryness under reduced pressure. The resulting crude crystals were recrystallized from ethyl acetate to give 10.4 g of 5-hydroxy-1-methylhydantoin (compound 1) in the form of white crystals.

m.p.: 135.0 - 136.0 °C

IR(KBr): 3180, 1750, 1715, 1446, 1115, 750 $cm^{-1}$

NMR(DMSO-$d_6$) $\delta$ = 2.72(s,3H), 4.98(d,1H,J = 8Hz), 6.88(d,1H,J = 8Hz), 10.74(br.s,1H)

Example 2.

Using a glyoxylic acid as a starting material, 5-hydroxy-3-methylhydantoin (compound 2) was obtained in the same way as Example 1.

m.p.: 115.5 - 116.5 °C

IR(KBr): 3350, 1765, 1700, 1465, 1068, 823 $cm^{-1}$

NMR(DMSO-$d_6$) $\delta$ = 2.80(s,3H), 5.16(d,1H,J = 8Hz), 6.72(d,1H,J = 8Hz), 8.61(s,1H)

Example 3.

19.0 g of glyoxyic acid monohydrate were dissolved in a mixture of 80 ml of 2-methoxyethanol and 150 ml of toluene in a 500 ml egg plant type flask fitted up with a Dean-Stark separator. Catalytic amounts of p-toluenesulfonic acid were added to the solution, and it was refluxed overnight. After the reaction mixture was evaporated to dryness under reduced pressure, 18.8 g of N-ethylurea was added to the residue, and 160 ml of acetic acid and 40 ml of water were further added thereto to dissolve them. The mixture was refluxed in oil bath for 2 hours, and then it was evaporated to dryness and acetic acid was removed by azeotropic distillation with toluene. The crude product was purified by column chromatography on silica gel (ethyl acetate), and was recrystallized from ethyl acetate to give 23 g of 5-hydroxy-1-ethylhydantoin (compound 3) in the form of white crystals.

m.p.: 119.0 - 120.0 °C

IR(KBr): 3340, 1778, 1702, 1470, 1102 $cm^{-1}$

NMR(DMSO-$d_6$) $\delta$=1.10(t,3H,J=7Hz), 3.14(dq,1H,$J_1$=14Hz,$J_2$=7Hz), 3.34(dq,1H,$J_1$=14Hz,$J_2$=7Hz), 5.09-(d,1H,J=9Hz), 6.88(d,1H,J=9Hz), 10.74(br.s,1H)

In the same way, the following compounds were obtained.

5-hydroxy-1-butylhydantoin (compound 4)
m.p.: 94.0 - 95.0 °C
IR(KBr): 3330, 1778, 1704, 1463, 1080, 750 $cm^{-1}$
NMR(DMSO-$d_6$) $\delta$=0.89(t,3H,J=7Hz), 1.2-1.4(m,2H), 1.4-1.6(m,2H), 3.0-3.4(m,2H), 5.06(d,1H,J=8Hz), 6.88-(d,1H,J=8Hz), 10.71(br.s,1H)

5-methoxy-1-methylhydantoin (compound 5)
m.p.: 95.0 - 96.0 °C
IR(KBr): 3160, 1764, 1718, 1442, 1080 $cm^{-1}$
NMR(DMSO-$d_6$) $\delta$ =2.79(s,3H), 3.21(s,3H), 5.09(s,1H), 11.05(br.s,1H)

5-butoxy-3-methylhydantoin (compound 6)
m.p.: 37.0 - 39.0 °C
IR(KBr): 3300, 2950, 1780, 1720, 1462, 1075 $cm^{-1}$
NMR(DMSO-$d_6$) $\delta$ =0.88(t,3H,J=7Hz), 1.2-1.5(m,2H), 1.4-1.6(m,2H), 2.83(s,3H), 3.4-3.6(m,2H), 5.21-(d,1H,J=2Hz), 8.84(br.s,1H)

Example 4.

5.0 g of 5-hydroxy-1-methylhydantoin (compound 1) was dissolved in absolute methanol and 6.7 g of triethylamine was added to the solution. 10.9 g (1.5 equivalent) of p-toluenesulfonyl chloride was then added to the solution, and was stirred at room temperature for 3 hours. After the reaction mixture was evaporated to dryness, a small amount of water was added to the residue and was extracted with ethyl acetate. The organic layer was dried over sodium sulfate and evaporated to dryness, and the residue was purified by column chromatography on silica gel (ethyl acetate). The obtained crude compound was recrystallized from a mixture of ethyl acetate and hexane to give 3.8 g of 5-methoxy-1-methylhydantoin (compound 5) in the form of white crystals.

In the same way, the following compounds were obtained.

5-ethoxy-1-methylhydantoin (compound 7)
m.p.: 96.0 - 97.0 °C
IR(KBr): 3160, 1765, 1720, 1443, 1115 $cm^{-1}$
NMR(DMSO-$d_6$) $\delta$ =1.15(t,3H,J=7Hz), 2.79(s,3H), 3.43(dq,1H,$J_1$=2Hz,$J_2$=7Hz), 3.56-(dq,1H,$J_1$=2Hz,$J_2$=7Hz), 5.08(s,1H), 11.00(br.s,1H)

5-methoxy-3-methylhydantoin (compound 8)
m.p.: 57.0 - 58.0 °C
IR(KBr): 3300, 1760, 1715, 1468, 1082 $cm^{-1}$
NMR(DMSO-$d_6$) $\delta$=2.83(s,3H), 3.26(s,3H), 5.12(d,1H,J=2Hz), 8.85(br.s,1H)
MS :$M^+$: 144, m/z: 116, 114, 86, 74, 59

5-methoxy-1-cyclohexylhydantoin (compound 9)
m.p.: 121.0 - 122.0 °C
IR(KBr): 3175, 3050, 2940, 2852, 1780, 1700, 1430, 1103, 768 $cm^{-1}$
NMR(DMSO-$d_6$) $\delta$ =0.9-1.35(m,3H), 1.35-1.7(m,3H), 1.7-1.9(m,4H), 3.18(s,3H), 3.56-(tt,1H,$J_1$=3.6Hz,$J_2$=12.0Hz), 5.25(s,1H), 11.0(br.s,1H)
MS :$M^+$: 212, m/z: 197, 182, 169, 131, 103, 98, 82, 67, 60, 55, 41

Example 5.

155 g of N-methylurea was added to 214 g of methyl pyruvate in a 3 l egg plant type flask. 800 ml of acetic acid and 200 ml of water were added to dissolve them and the solution was refluxed for 2.5 hours. Then, the reaction mixture was evaporated to dryness and acetic acid was removed by azeotropic distillation with toluene. The obtained crude product was purified by chromatography on silica gel (ethyl

acetate and 5% methanol/chloroform) to give 95 g of 5-hydroxy-1,5-dimethylhydantoin (compound 10).
m.p.: 122.0 - 123.0 °C
IR(KBr): 3340, 3200, 1780, 1765, 1720, 1440 $cm^{-1}$
NMR(DMSO-$d_6$)$\delta$ = 1.34(s,3H), 2.70(s,3H), 6.59(s,1H), 10.78(s,1H)
MS :$M^+$: 144, m/z: 129, 116, 73, 58, 43

In the same way, the following compound was obtained.

5-hydroxy-3,5-dimethylhydantoin (compound 11)
m.p.: (oily substance)
NMR(DMSO-$d_6$) $\delta$ = 1.37(s,3H), 2.81(s,3H), 6.50(s,1H), 8.63(s,1H)

Example 6.

21.0 g of glyoxylic acid benzyl ester benzyl alcoholate and 10.0 g of N-hexylurea were refluxed in 80% aqueous solution of acetic acid for 1.5 hours. After cooling, the reaction mixture was evaporated to dryness under reduced pressure. The obtained crude product was purified by column chromatography on silica gel (ethyl acetate : hexane = 1:1) and was recrystallized from benzene to give 1.4 g of 5-hydroxy-1-hexylhydantoin (compound 12) in the form of colorless needle crystals.
m.p.: 96.0 - 97.0 °C
IR(KBr): 3360, 3140, 2950, 1780, 1760, 1710, 1475, 1097, 755 $cm^{-1}$
NMR(DMSO-$d_6$) $\delta$ = 0.85(t,3H,J = 6.6Hz), 1.24(s,6H), 1.3-1.6(m,2H), 3.09-(ddd,1H,$J_1$ = 5.9Hz,$J_2$ = 8.2Hz,$J_3$ = 14.0Hz), 3.25(ddd,1H,$J_1$ = 7.3Hz,$J_2$ = 8.2Hz,$J_3$ = 14.0Hz), 5.04-(d,1H,J = 8.8Hz), 6.84(d,1H,J = 8.8Hz), 10.7(br.s,1H)
MS :$M^+$: 200, m/z: 129, 116, 89, 85, 58, 41, 30

In the same way, the following compounds were obtained.

5-hydroxy-1-decylhydantoin (compound 13)
m.p.: 100.0 - 101.0 °C
IR(KBr): 3350, 2920, 2855, 1780, 1760, 1710, 1470 1100, 755 $cm^{-1}$
NMR(DMSO-$d_6$) $\delta$ = 0.85(t,3H,J = 6.6Hz), 1.23(s,14H), 1.3-1.6(m,2H), 3.08-(ddd,1H,$J_1$ = 5.9Hz,$J_2$ = 8.1Hz,$J_3$ = 13.9Hz), 3.24(dt,1H,$J_1$ = 7.7Hz,$J_2$ = 13.9Hz), 5.03(d,1H,J = 8.8Hz), 6.84-(d,1H,J = 8.8Hz), 10.7(br.s,1H)
MS :$M^+$: 256, m/z; 129, 116, 99, 85, 69, 58, 41, 30

5-hydroxy-1-stearylhydantoin (compound 14)
m.p.: 112.0 - 114.0 °C
IR(KBr): 3320, 2910, 2850, 1780, 1760, 1710, 1480, 1095, 750 $cm^{-1}$
NMR(DMSO-$d_6$) $\delta$ = 0.84(t,3H,J = 6.6Hz), 1.23(s,30H), 1.4-1.6(m,2H), 3.08-(ddd,1H,$J_1$ = 6.1Hz,$J_2$ = 7.9Hz,$J_3$ = 14.0Hz), 3.24(dd,1H,$J_1$ = 6.4Hz,$J_2$ = 14.0Hz), 5.03(d,1H,J = 8.8Hz), 6.82-(d,1H,J = 8.8Hz), 10.68(br.s,1H)
MS :$M^+$: 368, m/z: 352, 296, 129, 113, 101, 69, 57, 43, 30

5-hydroxy-1-cyclopentylhydantoin (compound 15)
m.p.: 139.0 - 141.0 °C
IR(KBr): 3320, 2950, 2870, 1778, 1715, 1470, 1078, 763 $cm^{-1}$
NMR(DMSO-$d_6$)$\delta$ = 1.3-1.9(m,8H), 4.O0(tt,1H,$j_1$ = 8.1H,$J_2$ = 8.1Hz), 5.11(d,1H,j = 8.8Hz), 6.79(d,1H,J = 8.8Hz), 10.70(br.s,1H)
MS :$M^+$: 184, m/z: 155, 117, 99, 89, 84, 67, 56, 41, 27

5-hydroxy-1-cyclohexylhydantoin (compound 16)
m.p.: 176.0 - 178.0 °C
IR(KBr): 3310, 2920, 2850, 1770, 1700, 1450, 1115, 753 $cm^{-1}$
NMR(DMSO-$d_6$) $\delta$ = 0.9-1.4(m,3H), 1.4-1.9(m,7H) 3.51(tt,1H,$J_1$ = 4.0Hz,$J_2$ = 11.8Hz), 5.10(d,1H,J = 8.8Hz), 6.76(d,1H,J = 8.8Hz), 10.7(br.s,1H)
MS :$M^+$: 198, m/z: 155, 117, 82, 67, 56, 41, 27

5-hydroxy-1-(1,3-dimethylbutyl)hydantoin (compound 17)
m.p.: 148.0 - 149.0 °C

IR(KBr): 3250, 2950, 1762, 1700, 1445, 1100 $cm^{-1}$
NMR(DMSO-$d_6$) $\delta$ =0.7-1.0(m,6H), 1.1-1.2(m,3H), 1.2-1.7(m,3H), 3.8-4.0(m,1H), 5.06(d,J=8.8Hz), [5.10-(d,J=8.8Hz);total1H], 6.78(d,J=8.8Hz), [6.80(d,J=8.8Hz);total1H], 10.72(br.s,1H)
MS :$M^+$: 200, m/z: 143, 100, 72, 44, 43, 41

5-hydroxy-1-tert-butylhydantoin (compound 18)
m.p.: 189.0 - 190.5 °C
IR(KBr): 3230, 2965, 1760, 1710, 1436, 1230, 1080 $cm^{-1}$
NMR(DMSO-$d_6$) $\delta$ =1.37(s,9H), 5.14(s,1H), 6.78(br.s,1H), 10.47(br.s,1H)
MS :$M^+$: 172, m/z: 157, 84, 58, 41

5-hydroxy-1,3-dicyclohexylhydantoin (compound 19)
m.p.: 148.0 - 149.0 °C
IR(KBr): 3275, 2930, 2850, 1766, 1700, 1678, 1450, 1112, 760 $cm^{-1}$
NMR(DMSO-$d_6$) $\delta$ =0.9-1.4(m,6H), 1.4-1.8(m,12H) 1.8-2.0(m,2H), 3.54(tt,1H,$J_1$=4.0Hz,$J_2$=11.0Hz), 3.69-(tt,1H,$J_1$=3.8Hz,$J_2$=12.0Hz), 5.09(s,1H), 6.82(br.s,1H)
MS :$M^+$: 280, m/z: 237, 199, 181, 155, 117, 99, 83, 67, 55, 41

Example 7.

750 mg of glyoxylic acid methyl ester methyl alcoholate and 920 mg of N-cyclohexylurea were refluxed in 50 ml of a mixture of acetic acid and methanol (4:1) for 1 hour. After cooling, the solvent was removed by distillation and the residue was purified by column chromatography on silica gel. The obtained crude product was recrystallized from benzene to give 420 mg of 5-methoxy-3-cyclohexylhydantoin (compound 20) in the form of colorless needle crystals.
m.p.: 135.0 - 137.0 °C
IR(KBr): 3320, 2915, 2850, 1768, 1710, 1422, 1105 $cm^{-1}$
NMR(DMSO-$d_6$) $\delta$=0.9-1.4(m,3H), 1.4-1.8(m,4H), 1.8-2.1(m,3H), 3.22(s,3H), 3.70-(tt,1H,$J_1$=4.0Hz,$J_2$=14.0Hz), 5.09(s,1H), 8.80(br.s,1H)
MS :$M^+$: 182 m/z: 131, 99, 60, 55, 41

Example 8.

2.0 g of 5-hydroxy-1-cyclohexylhydantoin was dissolved in 30 ml of methanol containing 600 mg of sodium methoxide. 20 minutes thereafter, 0.7 ml of methyl iodide was added dropwise to the solution and the reaction was continued at 50 °C for 1 hour. The solvent was removed by distillation and the residue was purified by thin layer chromatography on silica gel (ethyl acetate : hexane = 1:1). The obtained crude product was recrystallized from benzene to give 500 mg of 5-hydroxy-1-cyclohexyl-3-methylhydantoin (compound 21) in the form of colorless needle crystals.
m.p.: 140.0 - 141.0 °C
IR(KBr): 3360, 2940, 2802, 1770, 1700, 1445, 1070, 762 $cm^{-1}$
NMR(DMSO-$d_6$) $\delta$ =0.9-1.4(m,3H), 1.4-1.9(m,7H), 2.81(s,3H), 3.55(tt,1H,$J_1$=4.0Hz,$J_2$=11.8Hz), 5.16-(d,1H,J=8.8Hz), 6.82(d,1H,J=8.8Hz)
MS :$M^+$: 212 m/z: 169, 131, 82, 67, 56, 41, 27
In the same way, the following compound was obtained.

5-hydroxy-1,3-dimethylhydantoin (compound 22)
m.p.: (colorless oily substance)
IR(KBr): 3300, 3000, 1776, 1715, 1195, 703 $cm^{-1}$
NMR(DMSO-$d_6$) $\delta$ =3.02(s,6H), 5.14(s,1H), 5.25(br.s,1H)
MS :$M^+$: 144 m/z: 127, 116, 88, 59, 42

The compound of the present invention may be produced by each of the above-mentioned preparations.

The following description serves to illustrate the pharmaceutical properties of compounds of the present invention.

(1) Acute toxicity test

The test compounds of the present invention were administered to groups of 10 ddY-strain male mice, and the $LD_{50}$ values were calculated based on the death rate for 7 days thereafter according to the Litchfield-Wilcoxon's method.

As a result, in every case of oral, intravenous, intraperitoneal and subcutaneous administrations, none of the mice, which were given more than 5,000 mg/kg of the test compounds, died. The mice also have no momentary symptom directly after the administration. Therefore the $LD_{50}$ values of the compounds of the present invention were more than 5,000 mg/kg. Furthermore, no abnormalities were found by autopsy after the medication in any organs.

(2) Hypoglycemic effect

Groups of 10 Sprague-Dawley-strain male rats (weighing about 250 g) which were fasted for 18 hours were used for measurement of hypoglycemic effect according to the modified method by Dulin et al. (Dulin, W. L. et al., Proc. Soc. Expl. Med., 107, 245(1961)). That is, 0.5 ml/100 g of 20% aqueous solution of glucose was subcutaneously administered to the backs of rats to prevent a decrease in blood glucose level because of the fasting. Immediately thereafter the test drug was orally given, and 2 hours later, the animals underwent laparotomy under pentobarbital anesthesia, then blood was drawn from the inferior vena cava. The obtained blood sample was allowed to stand for 30 minutes to complete the coagulation and was centrifuged to obtain the serum. Blood sugar level was measured according to the mutalotase GOD method (Trinder, Ann. Clin. Biochem., 6, 24(1979)).

An example of the results is shown in Table 1.

Table 1

| Test Compound | Dosage (mg/kg) | Blood Glucose Level (mg/dl) | Decrease (%) |
|---|---|---|---|
| control | - | 134.6 ± 2.8 | 0 |
| compound 1 | 25 | 89.0 ± 4.1 | 33.9 |
| | 50 | 87.1 ± 3.3 | 35.3 |
| | 200 | 81.8 ± 4.0 | 39.2 |
| compound 2 | 200 | 115.5 ± 3.9 | 14.2 |
| compound 3 | 200 | 76.0 ± 3.8 | 43.5 |
| compound 4 | 200 | 82.6 ± 3.6 | 38.6 |
| compound 5 | 200 | 109.4 ± 2.3 | 18.7 |
| compound 15 | 200 | 85.3 ± 5.9 | 36.6 |
| compound 16 | 200 | 89.6 ± 2.7 | 33.4 |
| compound 23 | 200 | 106.9 ± 3.9 | 20.6 |
| Tolubutamide | 50 | 63.4 ± 4.7 | 52.9 |
| | 100 | 53.0 ± 4.5 | 60.6 |

(compound 23 : 1-methylhydantoin)

(3) Hypolipidemic effect

The test drugs were orally administered to groups of 6 Wisterstrain male rats (weighing 210-250) which were fasted for 20 hours. 2 hours later, the serum was obtained in the same manner described in (2). Serum triglyceride was measured according to the GPO-p-chlorophenol colorimetric determination and serum free fatty acid was measured according to the Acyl CoA Synthetase-Acyl CoA Oxidase method.

An example of the results is shown in Table 2.

Table 2

| Test Compound | Dosage (mg/kg) | Serum Triglyceride Level (mg/dl) [Decrease (%)] | Serum Fatty Acid Level (mEq/dl) [Decrease (%)] |
|---|---|---|---|
| control | - | 33.5 ± 2.9 [0] | 0.51 ± 0.44 [0] |
| compound 1 | 100 | 10.7 ± 1.3 [68] | 0.17 ± 0.01 [66] |
| compound 2 | 100 | 13.8 ± 0.9 [59] | 0.28 ± 0.04 [45] |
| compound 4 | 100 | 15.3 ± 2.4 [54] | 0.30 ± 0.01 [41] |
| compound 23 | 100 | 18.5 ± 1.8 [45] | 0.29 ± 0.01 [43] |

(4) Diuretic effect

20 mg/kg of the compound 1 of the present invention was orally administered to rats, and the total amount of excreted sodium within 1 day was then measured according to the method by Lipschitz (J.Pharmacol. Exp. Ther. 79, 97 (1943)). As a result, the amount of excreted sodium on the group of rats which were given the compound 1 was 1.8 times as large as on the control group.

Further, in the case of subcutaneous administration to mice, up to 2 hours after the administration, the group which was given the compound 1 had 50.5% increase in the amount of urine compared with the control group.

In addition, 200 mg/kg of the compound 1 of the present invention was orally administered to glucose loaded rats and the urine sugar was measured. No difference was found between the medicated and control groups.

As clearly shown by the above-mentioned results, the compounds of the present invention have excellent hypoglycemic effect. Having an excellent feature of maintaining the subjects always at nearly the normal level without excessively lowering the blood sugar level even on high doses, the compounds of the present invention are extremely useful drugs for the treatment of severe hyperglycemia. As mentioned above, the compounds of the present invention have low toxicity and increased safety, so that longterm continuous administration and oral use are possible. Therefore, the compounds are not only useful as antidiabetics but also as drugs for various diseases associated with diabetes, e.g. diabetic angiopathy, diabetic arteriosclerosis, diabetic retinitis, diabetic nephropathy, diabetic neurosis and diabetic microangiopathy. Furthermore, the compounds of the present invention also have a diuretic effect, consequently they are very useful as drugs for the above-mentioned diseases.

In addition, as shown in Table 2, the compounds of the present invention have a hypolipidemic effect. Therefore, they are useful as drugs for the treatment of hyperlipidemia and for the treatment or the prevention of various diseases caused by hyperlipidemia, such as arteriosclerosis, obesity and cardiopathy.

The compounds of the present invention may be made up into pharmaceutical compositions by combination with appropriate pharmaceutically acceptable carriers or diluents, and may be formulated into preparations in solid, semisolid, liquid or gaseous form such as tablets, capsules, powders, granules, ointments, solutions, suppositories, injections, inhalants, aerosols and cataplasms in a conventional manner for oral or parenteral administrations.

In pharmaceutical dosage forms, the compounds of the present invention may be used in the form of their pharmaceutically acceptable salts, and also may be used alone or in appropriate association, as well as in combination with other pharmaceutically active components.

In case of oral preparations, the compounds of the present invention may be used alone or combined with appropriate additives to make tablets, powders, granules or capsules, e.g. with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethylcellulose; with lubricants such as talc or magnesium stearate; and if desired, with diluents, buffering agents, moistening agents, preservatives and flavoring agents.

The compounds of the invention may also be made into ointments by combination with ointment bases such as vaseline, paraffin, plastibase, simple ointment, hydrophilic ointment, hydrophilic vaseline and hydrophilic plastibase.

Furthermore, they may be made into suppositories by mixing with a variety of bases such as emulsifying bases or water-soluble bases.

The compounds of the present invention may be formulated into preparations for injection by dissolving, suspending or emulsifying them in aqueous or non-aqueous solvent, such as vegetable oil, synthetic aliphatic acid glycerides, esters of higher aliphatic acids or propylene glycol; and if desired, with conventional additives such as solubilizers, isotonic agents, suspending agents, emulsifying agents, stabilizers and preservatives.

In the case of inhalations or aerosol preparations, the compounds of the invention in the form of a liquid or minute powder may be filled up in an aerosol container with gas or liquid propellants agents, and if desired, with conventional adjuvants such as humidifying agents added. They may also be applied as pharmaceuticals for non-pressurized preparation such as in a nebulizer or an atomizer.

Cataplasms may be prepared by mixing the compounds with mentha oil, concentrated glycerin, kaolin or other suitable additives.

The desirable dose of the hydantoin derivatives of the present invention varies with the subject, drug form, method and period of administration. However, in order to obtain desirable effects, generally it is recommended to administer orally 1 to 1000 mg/kg, preferably 5 to 600 mg/kg daily. Unit preparations containing appropriate amounts of the compounds of the present invention are also recommended to administer by 1 to several units daily.

In case of parenteral administrations e.g. injections, doses of the compounds in the order of one tenth to one third of the above dose are preferable as daily doses.

Examples of pharmaceutical compositions containing the compounds of the present invention as active ingredients are given below:-

Prescription example 1 (tablet)

| Component | Content in a tablet (mg) |
| --- | --- |
| compound of the present invention | 100 |
| lactose | 130 |
| corn starch | 40 |
| magnesium stearate | 10 |
| Total | 280 mg |

Prescription example 2 (capsule)

| Component | Content in a capsule (mg) |
| --- | --- |
| Compound of the present invention | 50 |
| lactose | 250 |
| Total | 300 mg |

Prescription example 3 (injection)

| Component | Content in an ampule (mg) |
| --- | --- |
| Compound of the present invention | 10 |
| sodium chloride | proper amount |
| distilled water for injection | proper amount |
| Total | 1 ml |

Prescription example 4 (ointment)

| Component | Weight (g) |
| --- | --- |
| Compound of the present invention | 1 |
| emulsified wax | 30 |
| white petrolatum | 50 |
| liquid paraffin | 20 |
| Total | 101 g |

Prescription example 5 (suppository)

| Component | Content in a suppository (mg) |
| --- | --- |
| Compound of the present invention | 20 |
| cacao butter | 1980 |
| Total | 2000 mg |

**Claims**

**Claims for the following Contracting States: BE, CH, DE, FR, GB, IT, LI, LU, NL, SE**

**1.** A hydantoin derivative of the general formula I

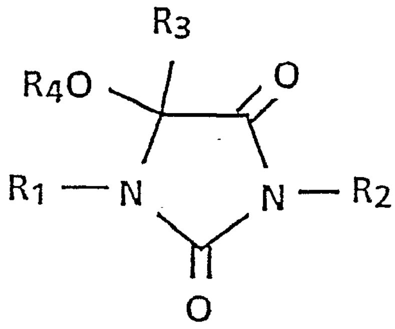

(I)

wherein $R_1$ represents a straight or branched chain alkyl group having from 2 to 20 carbon atoms or a cycloalkyl group having from 3 to 8 carbon atoms, each of $R_2$, $R_3$ and $R_4$, which may be the same or different, represents hydrogen or a straight or branched chain alkyl group having from 1 to 20 carbon atoms or a cycloalkyl group having from 3 to 8 carbon atoms,
and pharmaceutically acceptable salts thereof.

2. A hydantoin derivative according to claim 1, wherein $R_2$ and $R_3$ represent hydrogen atoms.

3. A hydantoin derivative according to claim 1 or claim 2, wherein $R_4$ represents a hydrogen atom.

4. A pharmaceutical composition containing a compound of the general formula (I):

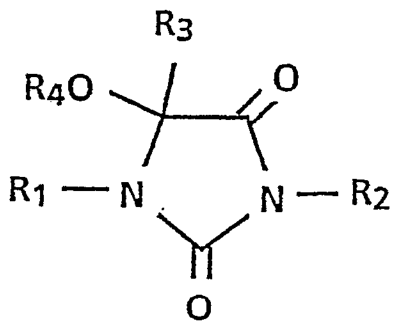

(I)

wherein each of $R_1$, $R_2$, $R_3$ and $R_4$, which may be the same or different, represents a hydrogen atom, an alkyl group having 1 to 20 carbon atoms or a cycloalkyl group having 3 to 8 carbon atoms;
and pharmaceutically acceptable salts thereof, together with a pharmaceutically acceptable carrier or diluent.

5. A pharmaceutical composition according to claim 4, wherein $R_2$ and $R_3$ represent a hydrogen atom.

6. A pharmaceutical composition according to claim 4 or claim 5, wherein $R_4$ represents a hydrogen atom.

7. A pharmaceutical composition according to any claims 4 to 6, wherein $R_1$ represent an alkyl group having 1 to 20 carbon atoms.

8. The use of hydantoin derivatives of the general formula (II):

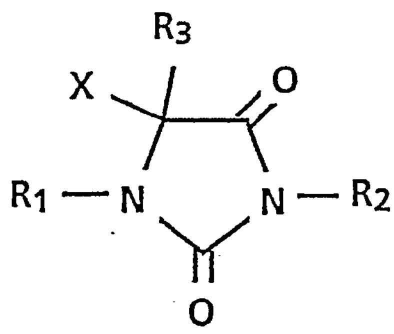

(II)

wherein X represents a hydrogen atom or a group of the formula $-OR_4$, and each of $R_1$, $R_2$, $R_3$ and $R_4$, which may be the same or different, represents a hydrogen atom, an alkyl group having 1 to 20 carbon atoms or a cycloalkyl group having 3 to 8 carbon atoms,
and pharmaceutically acceptable salts thereof, for the manufacture of hypoglycemic agents.

**9.** The use of hydantoin derivatives of the general formula (II):

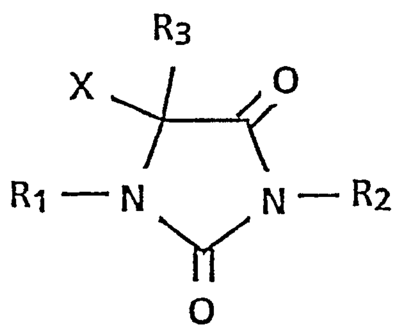

(II)

wherein X represents a hydrogen atom or a group of the formula $-OR_4$, and each of $R_1$, $R_2$, $R_3$ and $R_4$, which may be the same or different, represents a hydrogen atom, an alkyl group having 1 to 20 carbon atoms or a cycloalkyl group having 3 to 8 carbon atoms,
and pharmaceutically acceptable salts thereof, for the manufacture of hypolipidemic agents.

**10.** The use of hydantoin derivatives of the general formula (II):

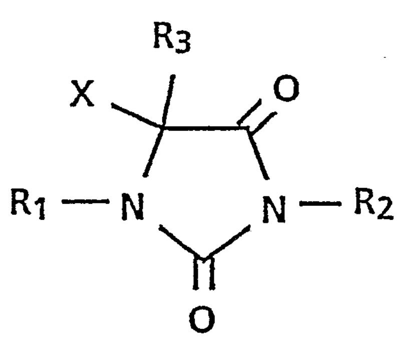

(II)

wherein X represents a hydrogen atom or a group of the formula $-OR_4$, and each of $R_1$, $R_2$, $R_3$ and $R_4$, which may be the same or different, represents a hydrogen atom, an alkyl group having 1 to 20 carbon atoms or a cycloalkyl group having 3 to 8 carbon atoms,
and pharmaceutically acceptable salts thereof, for the manufacture of diuretics.

**Claims for the following Contracting State: AT**

1. A process for the preparation of hydantoin derivatives of the general formula (I)

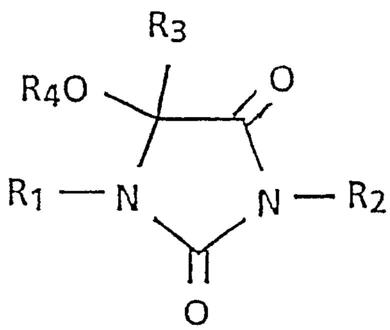

(I)

wherein $R_1$ represents a straight or branched chain alkyl group having from 2 to 20 carbon atoms or a cycloalkyl group having from 3 to 8 carbon atoms, and each of $R_2$, $R_3$ and $R_4$, which may be the same or different, represents hydrogen or a straight or branched chain alkyl group having from 1 to 20 carbon atoms or a cycloalkyl group having from 3 to 8 carbon atoms,
and pharmaceutically acceptable salts thereof, which comprises

(a) reacting a glyoxylic acid ester, or an O-alkylglyoxalic acid ester with an N-alkylurea, N-cycloalkylurea, N,N'-dialkylurea or N,N'-dicycloalkylurea,
(b) effecting an o-alkylation reaction on a hydantoin derivative or
(c) effecting an N-alkylation reaction on a hydantoin derivative.

2. A process according to claim 1 wherein $R_2$ and $R_3$ represent hydrogen atoms.

3. A process according to claim 1 or claim 2, wherein $R_4$ represents a hydrogen atom.

**Revendications**
**Revendications pour les Etats contractants suivants: BE, CH, DE, FR, GB, IT, LI, LU, NL, SE**

1. Dérivé d'hydantoine de la formule générale I

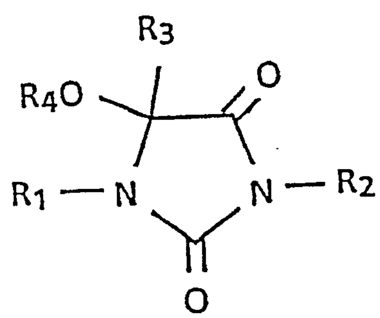

(I)

où $R_1$ représente un groupe alkyle en chaîne droite ou ramifiée comptant 2 à 20 atomes de carbone ou un groupe cycloalkyle comptant 3 à 8 atomes de carbone, $R_2$, $R_3$ et $R_4$, qui peuvent être identiques ou différents, représentent chacun hydrogène ou un groupe alkyle en chaîne droite où ramifiée comptant 1 à 20 atomes de carbone ou un groupe cycloalkyle comptant 3 à 8 atomes de carbone,
et les sels pharmaceutiquement acceptables de celui-ci.

2. Dérivé d'hydantoine suivant la revendication 1 où $R_2$ et $R_3$ représentent atomes d'hydrogène.

3. Dérivé d'hydantoine suivant la revendication 1 ou 2 où $R_4$ représente un atome d'hydrogène.

4. Composition pharmaceutique contenant un constituant de la formule générale (I):

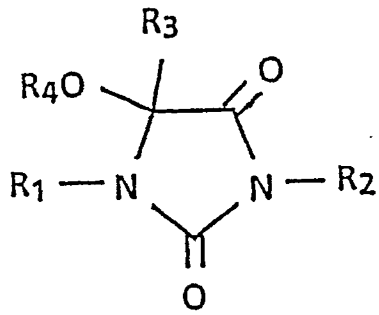

(I)

où $R_1$, $R_2$, $R_3$ et $R_4$, qui peuvent être identiques ou différents, représentent chacun un atom d'hydrogène, un groupe alkyle comptant 1 à 20 atomes de carbone ou un groupe cycloalkyle comptant 3 à 8 atomes de carbone;
et les sels pharmaceutiquement acceptables de celui-ci, avec un porteur ou diluant pharmaceutiquement acceptable.

5. Composition pharmaceutique suivant la revendication 4 où $R_2$ et $R_3$ représentent un atome d'hydrogène.

6. Composition pharmaceutique suivant la revendication 4 ou 5 où $R_4$ représente un atome d'hydrogène.

7. Composition pharmaceutique suivant l'une quelconque des revendications 4 à 6 où $R_1$ représente un groupe alkyle comptant 1 à 20 atomes de carbone.

8. Utilisation de dérivés d'hydantoine de la formule générale (II):

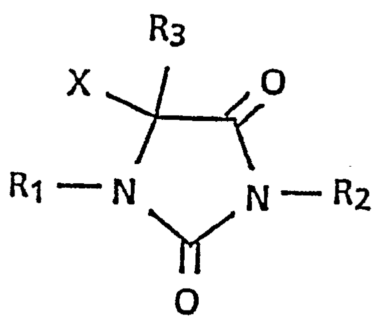

(II)

où X représente un atome d'hydrogène ou un groupe de la formule $-OR_4$, et où $R_1$, $R_2$, $R_3$ et $R_4$, qui peuvent être identiques ou différents, représentent chacun un atome d'hydrogène, un groupe alkyle comptant 1 à 20 atomes de carbone ou un groupe cycloalkyle comptant 3 à 8 atomes de carbone,
et les sels pharmaceutiquement acceptables de celui-ci, pour la préparation d'agents hypoglycémiants.

9. Utilisation de dérivés d'hydantoine de la formule générale (II):

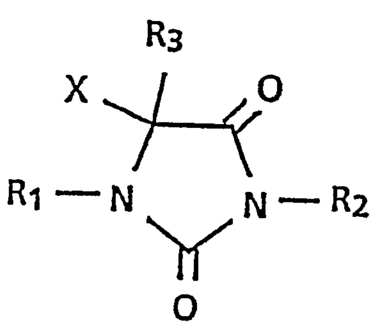

(II)

où X représente un atome d'hydrogène ou un groupe de la formule $-OR_4$, et où $R_1$, $R_2$, $R_3$ et $R_4$, qui peuvent être identiques ou différents, représentent chacun un atome d'hydrogène, un groupe alkyle comptant 1 à 20 atomes de carbone ou un groupe cycloalkyle comptant 3 à 8 atomes de carbone, et les sels pharmaceutiquement acceptables de celui-ci, pour la préparation d'agents hypolipidémiants.

**10.** Utilisation de dérivés d'hydantoine de la formule générale (II):

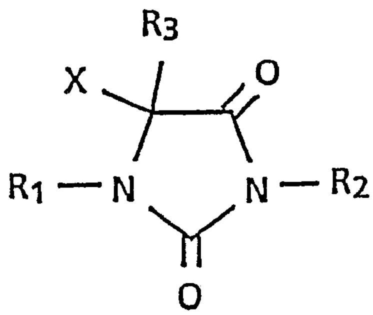

(II)

où X représente un atome d'hydrogène ou un groupe de la formule $-OR_4$, et où $R_1$, $R_2$, $R_3$ et $R_4$, qui peuvent être identiques ou différents, représentent chacun un atome d'hydrogène, un groupe alkyle comptant 1 à 20 atomes de carbone ou un groupe cycloalkyle comptant 3 à 8 atomes de carbone, et les sels pharmaceutiquement acceptables de celui-ci, pour la préparation des diurétiques.

**Revendications pour l'Etat contractant suivant: AT**

**1.** Procédé de préparation d'un dérivé d'hydantoine de la formule générale (I)

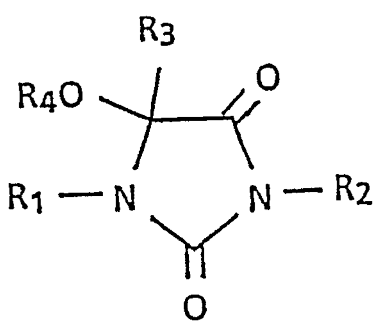

(I)

où $R_1$ représente un groupe alkyle en chaîne droite ou ramifiée comptant 2 à 20 atomes de carbone ou un groupe cycloalkyle comptant 3 à 8 atomes de carbone, $R_2$, $R_3$ et $R_4$, qui peuvent être identiques ou différents, représentent chacun hydrogène ou un groupe alkyle en chaîne droite où ramifiée comptant 1 à 20 atomes de carbone ou un groupe cycloalkyle comptant 3 à 8 atomes de carbone,
et les sels pharmaceutiquement acceptables de celui-ci, qui contient
(a) laisser réagir un ester de l'acide glyoxylique ou un ester de l'acide O-alkyleglyoxalique avec une N-alkyleurée, N-cycloalkyleurée, N,N'-dialkyleurée ou N,N'-dicycloalkyleurée,
(b) laisser faire une réaction O-alcylation sur un dérivé d'hydantoine ou
(c) laisser faire une réaction N-alcylation sur un dérivé d'hydantoine.

**2.** Procédé suivant la revendication 1 où $R_2$ et $R_3$ représentent atomes d'hydrogène.

**3.** Procédé suivant la revendication 1 ou 2 où $R_4$ représente un atome d'hydrogène.

**Patentansprüche**
**Patentansprüche für folgende Vertragsstaaten: BE, CH, DE, FR, GB, IT, LI, LU, NL, SE**

1. Hydantoinderivat der allgemeinen Formel I

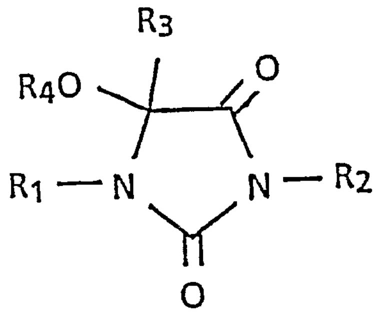

(I)

worin $R_1$ eine gerade oder verzweigte Alkylgruppe mit 2 bis 20 Kohlenstoffatomen bedeutet oder eine Cycloalkylgruppe mit 3 bis 8 Kohlenstoffatomen, wobei jeder von $R_2$, $R_3$ und $R_4$, die gleich oder verschieden sein können, Wasserstoff oder eine gerade oder verzweigte Alkylgruppe mit 1 bis 20 Kohlenstoffatomen oder eine Cycloalkylgruppe mit 3 bis 8 Kohlenstoffatomen bedeutet,
und pharmazeutisch annehmbare Salze davon.

2. Hydantoinderivat nach Anspruch 1, worin $R_2$ und $R_3$ Wasserstoffatome bedeuten.

3. Hydantoinderivat nach Anspruch 1 oder Anspruch 2, worin $R_4$ ein Wasserstoffatom bedeutet.

4. Pharmazeutische Zusammensetzung, die einen Wirkstoff der allgemeinen Formel (I) enthält:

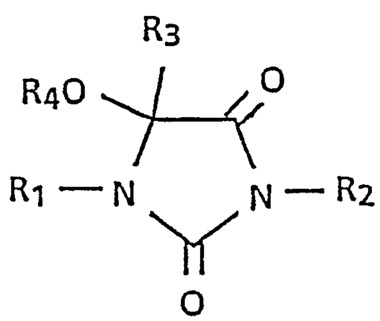

(I)

worin jeder von $R_1$, $R_2$, $R_3$ und $R_4$, welche gleich oder verschieden sein können, ein Wasserstoffatom, eine Alkylgruppe mit 1 bis 20 Kohlenstoffatomen oder eine Cycloalkylgruppe mit 3 bis 8 Kohlenstoffatomen bedeutet;
und pharmazeutisch annehmbare Salze davon, zusammen mit einem pharmazeutisch annehmbaren Träger oder Verdünnungsmittel.

5. Pharmazeutische Zusammensetzung nach Anspruch 4, worin $R_2$ und $R_3$ ein Wasserstoffatom bedeuten.

6. Pharmazeutische Zusammensetzung nach Anspruch 4 oder Anspruch 5, worin $R_4$ ein Wasserstoffatom bedeutet.

7. Pharmazeutische Zusammensetzung nach einem der Ansprüche 4 bis 6, worin $R_1$ eine Alkylgruppe mit 1 bis 20 Kohlenstoffatomen bedeutet.

8. Verwendung von Hydantoinderivaten der allgemeinen Formel (II):

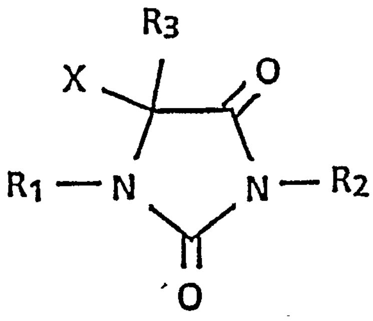

(II)

worin X ein Wasserstoffatom oder eine Gruppe der Formel $-OR_4$ bedeutet, und jeder von $R_1$, $R_2$, $R_3$ und $R_4$, welche gleich oder verschieden sein können, ein Wasserstoffatom, eine Alkylgruppe mit 1 bis 20 Kohlenstoffatomen oder eine Cycloalkylgruppe mit 3 bis 8 Kohlenstoffatomen bedeutet,
und pharmazeutisch annehmbare Salze davon, für die Herstellung von hypoglykämischen Mitteln.

**9.** Verwendung von Hydantoinderivaten der allgemeinen Formel (II):

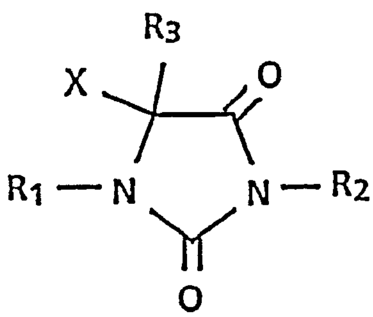

(II)

worin X ein Wasserstoffatom oder eine Gruppe der Formel $-OR_4$ bedeutet, und jeder von $R_1$, $R_2$, $R_3$ und $R_4$, welche gleich oder verschieden sein können, ein Wasserstoffatom, eine Alkylgruppe mit 1 bis 20 Kohlenstoffatomen oder eine Cycloalkylgruppe mit 3 bis 8 Kohlenstoffatomen bedeutet,
und pharmazeutisch annehmbare Salze davon, für die Herstellung von hypolipidämischen Mitteln.

**10.** Verwendung von Hydantoinderivaten der allgemeinen Formel (II):

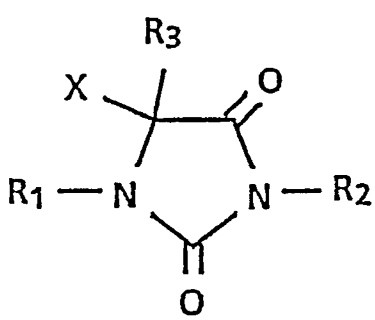

(II)

worin X ein Wasserstoffatom oder eine Gruppe der Formel $-OR_4$ bedeutet, und jeder von $R_1$, $R_2$, $R_3$ und $R_4$, welche gleich oder verschieden sein können, ein Wasserstoffatom, eine Alkylgruppe mit 1 bis 20 Kohlenstoffatomen oder eine Cycloalkylgruppe mit 3 bis 8 Kohlenstoffatomen bedeutet,
und pharmazeutisch annehmbare Salze davon, für die Herstellung von Diuretika.

**Patentansprüche für folgenden Vertragsstaat: AT**

**1.** Verfahren zur Herstellung von Hydantoinderivaten der allgemeinen Formel (I)

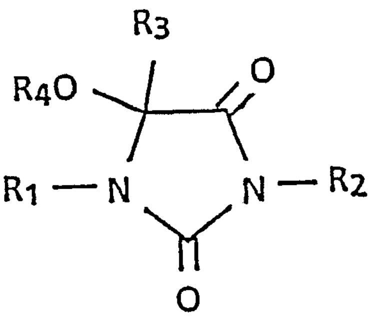

(I)

worin $R_1$ eine gerade oder verzweigte Alkylgruppe mit 2 bis 20 Kohlenstoffatomen oder eine Cycloalkylgruppe mit 3 bis 8 Kohlenstoffatomen bedeutet, und jeder von $R_2$, $R_3$ und $R_4$, welche gleich oder verschieden sein können, Wasserstoff oder eine gerade oder verzweigte Alkylgruppe mit 1 bis 20 Kohlenstoffatomen oder eine Cycloalkylgruppe mit 3 bis 8 Kohlenstoffatomen bedeutet,
und pharmazeutisch annehmbare Salze davon, welche umfassen

(a) Reagieren eines glyoxylischen Säureesters oder eines O-alkylglyoxalischen Säureesters mit einem N-Alkylharnstoff, N-Cycloalkylharnstoff, N,N'-Dialkylharnstoff oder N,N'-Dicycloalkylharnstoff,

(b) Ausführen einer O-Alkylierungsreaktion auf ein Hydantoinderivat oder

(c) Ausführen einer N-Alkylierungsreaktion auf ein Hydantoinderivat.

**2.** Verfahren nach Anspruch 1, worin $R_2$ und $R_3$ Wasserstoffatome bedeuten.

**3.** Verfahren nach Anspruch 1 oder Anspruch 2, worin $R_4$ ein Wasserstoffatom bedeutet.